# EUROPEAN PATENT APPLICATION

(11) **EP 4 011 860 A2**
(43) Date of publication of application: **15.06.2022**
(21) Application number: 21460047.0
(22) Date of filing: 21.12.2021
(51) Int. Cl.: C07C 67/08, C07C 67/54, C07C 69/24, C07C 69/58

(54) **METHOD FOR PREPARING FATTY ACID ESTERS**

(30) Priority: 22.12.2020 PL 43644220
(71) Applicant: Politechnika Slaska, 44-100 Gliwice (PL)
(72) Inventor: Brzeczek-Szafran, Alina, 40-018 Katowice (PL); Chrobok, Anna, 42-674 Zbroslawice (PL); Wieclawik, Justyna, 42-260 Rudnik (PL)

(57) **Abstract**

Fatty acid esters are prepared as follows: the fatty acid is reacted with alcohol in a molar ratio of 1:1-20:1 in the presence of mono, di- tri-, tetra-, penta- or hexacationic protic ionic liquids as catalyst used in a molar ratio of 0.1-300% with respect to the acid. The reaction occurs at 0-150 °C between 1 min and 24 h, preferably 1 hour. The formed ester is separated from the reaction mixture, and unreacted alcohol and water are removed by distillation from the ionic liquid phase, which can be reused.

## Description

The following describes a method for the preparation of fatty acid esters in the presence of protic ionic liquids.

Fatty acid esters have wide applications in the cosmetic, pharmaceutical, agrochemical and petrochemical industry, as lubricants, softeners, low-foaming agents and plasticizers.

Furthermore, methyl and ethyl esters of fatty acids are used as biodiesel for diesel engines with physical and chemical parameters similar to that of conventional diesel oil. Compared to other diesel fuels, biodiesel is sulfur-free, contributes to reduced emissions of pollutants during combustion, and biodegradable and safe under storage and transport conditions.

Currently, fatty acids esters are obtained on scale via esterification of long chain fatty acids or transesterification of triacylglycerols using acid or base catalysts. The preparation of fatty acid esters by base catalysis can be carried out under mild reaction conditions (temperatures 50-80 °C) and with low molar excess of alcohol, e.g. 5:1, however, issues arise during cleaning of the product from soaps and catalysts (Luque et al. Energy Environ. Sci., 2010, 3, 1706-1721 ). In the case of acid catalysis, it avoids soap formation but the utilization of mineral acids such as H₂SO₄ and HF, although very effective and cheap, are problematic during purification of the reaction mixture from catalysts that are miscible with reactants.

Numerous patents have described the preparation of fatty acid esters using mineral acids.

Patent CN101225324A discloses a method for the preparation of fatty acid esters using 1-2% by weight of H₂SO₄ in relation to the substrate. The process is conducted under 0.2-0.3 MPa pressure, at 80-95 °C for 16-20 h, and using a molar ratio of alcohol to oil of 0.8:1-1.2:1. After the process, the reaction mixture forms 2 phases: the upper phase mainly contains the product, which is washed with water, dried in *vacuo,* and distilled. The lower phase mainly contains glycerol and H₂SO₄. However, the patent fails to detail any further treatment of the lower phase.

In order to ensure the possible reusability of the catalyst, via separation from the reaction mixture, heterogeneous catalysts (solid or liquid) are used (Su, Guo, Green Chem., 2014, 16, 2934). The minimization of waste is very important in process development design according to the principles of green chemistry and sustainable development.

An important group of such catalysts are ionic liquids with Bronsted acidity, which, apart from high thermal stability and low vapor pressure, are characterized by the possibility of creating a separate phase in the reaction system during the process or after its completion.

Processes for producing biodiesel using ionic liquids as catalysts are the subject of numerous patents.

Patent TW201120207A describes a method for the preparation of palmitic, stearic, oleic, linoleic and myristic acid methyl esters using a sulfonic ionic liquid with a imidazolium, pyridinium or alkylammonium based cation. The esterification process is carried out at 25-120 °C for 0.1-10 h, with a 1:30 molar ratio of methanol to acid, where the molar ratio of the catalyst to the acid is 0.01-1.0. After the process was completed, methanol and water are distilled off, and the ionic liquid separated from the product, which can be reused.

Various types of sulfonic ionic liquids with Brønsted acidity are mentioned in various patents, which catalyze the esterification of fatty acids, or transesterification of triacylglycerols, and easily separate after the process and can be recycled (CN105132187A, US2011245522A1, CN1737086A, CN100491503C).

The disadvantage of sulfonic ionic liquids is their multistep synthesis, which increases the cost of the entire process. Their synthesis involves the preparation of a functionalized cation through the reaction of a tertiary aliphatic or aromatic amine and 1,3-propane sultone to obtain zwitterions, followed by acidification with Brønsted acid.

Protic ionic liquids, an alternative to sulfonic ionic liquids, are obtained by proton transfer from Brønsted acid to Brønsted base. Protic ionic liquids are characterized by simple synthesis and low reagent cost. The acidity of systems can be fine-tuned using acid in various molar ratios e.g. two- or three-fold excess of H₂SO₄ with respect to monoamine (1-methylimidazole, triethylamine, N-methylpyrrolidine) (Matuszek et al. Green Chem., 2014, 16, 3463-3471).

The patent literature describes the importance of using protic ionic liquids based on monoamines in the synthesis of esters on industrial scale. The esters include: succinic acid esters known from the Polish patent PL234515, phthalic and terephthalic acid esters known from the Polish patent PL234516, and levulinic acid esters known from the patent PL232790.

The aim of the invention is to develop an economical and effective method for the synthesis of fatty acid esters using di-, tri-, tetra-, penta- or hexacationic protic ionic liquid as catalyst. The prepared esters can be used, for example, as biodiesel. Polycationic protic ionic liquids obtained using excess amounts of H₂SO₄ are an attractive alternative to protic monocationic ionic liquids obtained via monoamines due to the simple synthetic method and lower production cost.

Our research has shown that it is possible to increase the efficiency of fatty acid ester synthesis by using di-, tri-, tetra-, penta- or hexacationic protic ionic liquid as catalyst, allowing the possibility of easy separation of the product from the catalyst and recycling of the catalyst in the process.

The proposed method for the preparation of fatty acid esters, involves the reaction of alcohol in a molar ratio from 1:1 to 20:1 in the presence of mono, di- tri-, tetra-, penta- or hexacationic protic ionic liquids as catalyst in a molar ratio of 0.1-300% in relation of the acid. The reaction occur at 0-150 °C, between 1 min and 24 h, preferably after 1 h. The formed ester is separated from the reaction mixture, while unreacted alcohol and water are removed by distillation from the ionic liquid phase, which can be reused.

The preferred fatty acid in the invention is oleic acid, palmitic acid, stearic acid, lauric acid, linoleic acid or myristic acid.

The preferred aliphatic alcohol is C1-C9 in the invention.

The preferred mono, di- tri-, tetra-, penta- or hexacationic protic ionic liquid in the invention is synthesized with a molar ratio of 1 amine group to sulfuric acid ranging between 1:1-1:5.

The preferred amine in the invention is monoamine with a general formula of NR₁R₂R₃, diamine with a general formula of NR₁R₂R₃NR₄R₅, triamine with a general formula of NR₁R₂R₃NR₄R₅NR₆R₇, tetraamine with a general formula of NR₁R₂R₃NR₄R₅NR₆R₇NR₈R₉, pentaamine with a general formula of NR₁R₂R₃NR₄R₅NR₆R₇NR₈R₉NR₁₀R₁₁, and hexaamine with a general formula of NR₁R₂R₃NR₄R₅NR₆R₇NR₈R₉NR₁₀R₁₁NR₁₂R₁₃, where R₁₋₁₃=H, C₁-C₂₀

The preferred amine in the invention is *N,N*-dimethylaminopropylamine, hexamethylenediamine, diethylenetriamine, bis(hexamethylene)triamine, triethylenetetramine, tetraethylenepentamine, pentaethylenehexamine.

The advantage of the invention is that it allows the synthesis of fatty acid esters at low temperatures, short reaction time and with 100% acid conversion. Additionally, the product is easily separated by phase separation. The final product is clean, does not require neutralization and is not contaminated with the catalyst. Furthermore, the current need for large volumes of water during separation and purification of the product has been eliminated. After separation of the product and removal of unreacted alcohol and water by distillation, the protic ionic liquid can be recycled. Ionic liquids used are characterized by low cost and simple preparation procedure.

The present invention is further illustrated with reference to the following preparation methods:

### Example 1

### Method for the synthesis of protic ionic liquid based on hydrogen sulfate anion and bis(hexamethylene)triammonium cation

Sulfuric acid (98%, 201 g) was placed into a 500 mL round bottom flask and stirred in an ice bath. Bis(hexamethylene)triamine (49 g, 227.5 mmol) was added dropwise with 1:9 molar ratio of amine to acid. The reaction mixture was stirred for 1 h at 80 °C until a homogeneous dark brown mixture was obtained. The prepared ionic liquid was dried under reduced pressure of 100 Pa at 50 °C for 8 h, giving the product in 98% yield.

### Example 2

### Method for the synthesis of protic ionic liquid based on hydrogen sulfate anion and N,N-dimethylaminopropylammonium cation

Sulfuric acid (98%, 213 g) was placed into a 500 mL round bottom flask and stirred in an ice bath. *N,N-*Dimethylaminopropylamine (37 g, 362.1 mmol) was added dropwise with 1:6 molar ratio of amine to acid. The reaction mixture was stirred for 1 h at 70 °C until a homogeneous dark brown mixture was obtained. The prepared ionic liquid was dried under reduced pressure of 100 Pa at 50 °C for 8 h, giving the product in 98% yield.

### Example 3

### Method for the synthesis of methyl oleate

90% oleic acid (20 g, 63.7 mmol), methanol (16.3 g, 509.8 mmol) and the prepared protic ionic liquid based on hydrogen sulfate anion and bis(hexamethylene)triammonium cation (3.5 g. 3.2 mmol), with a 1:9 molar ratio of amine to acid, was placed into a 100 mL round-bottomed flask equipped with a reflux condenser. The mixture was stirred 60 °C for 1 h, and then the phases in the reaction mixture are separated. Methyl oleate (the upper phase) was obtained in 99% yield. The lower layer contained the ionic liquid, which was washed with hexane, and unreacted methanol and water were removed by distillation. The purified ionic liquid was recycled.

### Example 4

### Method for the synthesis of methyl palmitate

99% palmitic acid (20 g, 77.2 mmol), methanol (24.7 g, 772.2 mmol) and the prepared protic ionic liquid based on hydrogen sulfate anion and bis(hexamethylene)triammonium cation (4.2 g, 3.9 mmol), with (1:9) molar ratio of amine to acid, were placed introduced into a 100 mL round-bottomed flask. The mixture was stirred at 40 °C for 3 h, then the phases were separated. Methyl palmitate (the upper phase) was obtained in 97% yield. The lower layer contained the ionic liquid, which was washed with hexane, and unreacted methanol and water removed by distillation. The purified ionic liquid was reused.

### Example 5

### Method for the synthesis of ethyl stearate

95% stearic acid (20 g, 66.8 mmol), ethanol (24.6 g, 534.3 mmol) and the prepared protic ionic liquid based on hydrogen sulfate anion and bis(hexamethylene)triammonium cation (2.2 g, 2.0 mmol), with (1:9) molar ratio of amine to acid, were placed into a 100 mL round-bottomed flask. The mixture is stirred at 60 °C for 1 h, and then the phases in the reaction mixture were separated. Ethyl stearate (the upper phase) was obtained in 99% yield. The lower layer contained the ionic liquid, which was washed with hexane, and unreacted ethanol and water were removed by distillation. The purified ionic liquid was reused.

### Example 6

### Method for the synthesis of butyl laureate

98% lauric acid (20 g, 97.8 mmol), butanol (58.0 g, 782.7 mmol) and the prepared protic ionic liquid based on hydrogen sulfate anion and *N,N-*dimethylaminopropylammonium cation (3.4 g, 4.9 mmol), with (1:6) molar ratio of amine to acid, was placed into a 100 mL round-bottomed flask. The mixture was stirred 60 °C for 1 h, and then the phases in the reaction mixture were separated. Butyl laureate (the upper phase) was obtained in 95% yield. The lower layer contained the ionic liquid, which is washed with hexane, and unreacted butanol and water were removed by distillation. The purified ionic liquid can be reused.

## Claims

1. Fatty acid esters are prepared as follows: the fatty acid is reacted with alcohol in a molar ratio of 1:1-20:1 in the presence of mono, di- tri-, tetra-, penta- or hexacationic protic ionic liquids as catalyst used in a molar ratio of 0.1-300% with respect to the acid. The reaction occurs at 0-150 °C between 1 min and 24 h, preferably 1 hour. The formed ester is separated from the reaction mixture, and unreacted alcohol and water are removed by distillation from the ionic liquid phase, which can be reused.

2. As stated in claim 1, the fatty is oleic acid, palmitic acid, stearic acid, lauric acid, linoleic acid or myristic acid.

3. As stated in claim 1, the alcohol is aliphatic alcohol C1-C9.

4. According to claim 1, mono, di- tri-, tetra-, penta- or hexacationic protic ionic liquid is the synthesized protic ionic liquid with molar ratio of amine group to sulfuric acid ranging from 1:1 to 1:5.

5. The amine described in claim 4 is a monoamine with a general formula of NR₁R₂R₃, diamine with a general formula of NR₁R₂R₃NR₄R₅, triamine with a general formula of NR₁R₂R₃NR₄R₅NR₆R₇, tetraamine with a general formula of NR₁R₂R₃NR₄R₅NR₆R₇NR₈R₉, pentaamine with a general formula of NR₁R₂R₃NR₄R₅NR₆R₇NR₈R₉NR₁₀R₁₁, and hexaamine with a general formula of NR₁R₂R₃NR₄R₅NR₆R₇NR₈R9NR₁₀R₁₁NR₁₂R₁₃, where R₁₋₁₃=H, C₁-C₂₀

6. The amine described in claim 4 is *N,N*-dimethylaminopropylamine, hexamethylenediamine, diethylenetriamine, bis(hexamethylene)triamine, triethylenetetramine, tetraethylenepentamine, or pentaethylenehexamine.
